(19) Europäisches Patentamt / European Patent Office / Office européen des brevets

(11) **EP 4 334 896 B1**

(12) **FASCICULE DE BREVET EUROPEEN**

(45) Date de publication et mention
de la délivrance du brevet:
**06.05.2026 Bulletin 2026/19**

(21) Numéro de dépôt: **22724823.4**

(22) Date de dépôt: **04.05.2022**

(51) Classification Internationale des Brevets (IPC):
**G06T 7/00** (2017.01)    **G06T 7/155** (2017.01)

(52) Classification Coopérative des Brevets (CPC):
**G06T 7/0012; G06T 7/155;** G06T 2207/30068;
G06T 2207/30096

(86) Numéro de dépôt international:
**PCT/FR2022/050865**

(87) Numéro de publication internationale:
**WO 2022/234235 (10.11.2022 Gazette 2022/45)**

(54) **PROCÉDÉ DE TRAITEMENT MORPHOLOGIQUE D'IMAGES RADAR MICRO-ONDES DANS LE DOMAINE MÉDICAL UTILISANT DIFFÉRENTES HYPOTHÈSES SUR LE MILIEU TRAVERSÉ PAR LES SIGNAUX MICRO-ONDES**

VERFAHREN ZUR MORPHOLOGISCHEN VERARBEITUNG VON MIKROWELLENRADARBILDERN IM MEDIZINISCHEN BEREICH UNTER VERWENDUNG VERSCHIEDENER HYPOTHESEN AUF DEM MEDIUM, DURCH DAS DIE MIKROWELLENSIGNALE GEHEN

METHOD FOR MORPHOLOGICAL PROCESSING OF MICROWAVE RADAR IMAGES IN THE MEDICAL FIELD USING DIFFERENT HYPOTHESES ON THE MEDIUM THROUGH WHICH THE MICROWAVE SIGNALS PASS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.05.2021 FR 2104690**

(43) Date de publication de la demande:
**13.03.2024 Bulletin 2024/11**

(73) Titulaire: **MVG Industries
91140 Villejust (FR)**

(72) Inventeurs:
• **FASOULA, Agathi
91140 VILLEJUST (FR)**
• **DUCHESNE, Luc
91140 VILLEJUST (FR)**

(74) Mandataire: **Regimbeau
20, rue de Chazelles
75847 Paris Cedex 17 (FR)**

(56) Documents cités:
**CN-A- 111 067 524     US-A1- 2005 107 692
US-A1- 2013 018 591     US-A1- 2019 175 095**

• **FASOULA A ET AL: "Super-resolution radar imaging for breast cancer detection with microwaves: the integrated information selection criteria", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 1868 - 1874, XP033625840, [retrieved on 20191002], DOI: 10.1109/ EMBC.2019.8857924**

EP 4 334 896 B1

**Description**

DOMAINE TECHNIQUE

L'invention concerne le domaine de l'imagerie médicale utilisant les ondes électromagnétiques dans la bande des fréquences micro-ondes et concerne plus particulièrement l'imagerie médicale pour l'analyse de tissus ou d'organes humains perméables aux ondes électromagnétiques. Et l'invention trouve notamment application dans l'imagerie du sein et la détection des pathologies mammaires.

ETAT DE LA TECHNIQUE

Les techniques d'imagerie micro-ondes permettent d'imager des organes humains perméables aux ondes électromagnétiques et sont des techniques prometteuses dans le domaine de l'imagerie du sein et la détection de pathologies telle que les cancers du sein.

L'imagerie micro-ondes met en œuvre des sondes d'émission configurées pour éclairer tout ou partie de l'organe à imager au moyen d'ondes électromagnétiques. Les ondes émises traversent la zone à imager et sont reçues par des sondes de réception. Les sondes peuvent, de manière complémentaire, être configurées pour émettre et recevoir simultanément. Les ondes reçues ont traversé la zone à imager en ayant subi des réflexions sur les obstacles rencontrés, aux endroits des contrastes diélectriques (par exemples une lésion cancéreuse située dans des tissus sains). L'ensemble des coefficients de transmission ainsi mesurés entre les sondes émettrices et les sondes réceptrices forment une acquisition multistatique. Ces acquisitions multistatiques servent d'entrée à des modules de traitement d'imagerie radar et permettent d'obtenir une image radar 2D ou 3D de l'organe ou d'une partie de l'organe.

Pour obtenir des images représentant au mieux la zone à imager il est nécessaire de connaitre a priori le milieu diélectrique le long des différents chemins parcourus par les ondes électromagnétiques entre les sondes d'émission, chaque point de la zone à imager considérée, et les sondes de réception.

Or cette connaissance a priori des propriétés diélectriques des organes à imager n'est pas accessible et nécessite de faire des hypothèses sur le milieu traversé ce qui conduit à des images qui peuvent être de mauvaise qualité.

Le document US2013/018591 A1 (GRZEGORCZYK TOMASZ M [US]) (2013-01-17) divulgue un appareil d'imagerie par micro-ondes utilisant un faisceau d'antennes fonctionnant pour recueillir des informations de champ électromagnétique pour un matériau soumis à un examen par imagerie. Un procédé et un dispositif de traitement d'images utilisent l'approximation dipolaire discrète (DDA, de l'anglais discrete dipole approximation) et réduisent de façon drastique le temps nécessaire au traitement des données mesurées et à l'estimation des propriétés du matériau interrogé. Une évaluation initiale des propriétés du matériau est effectuée et le champ obtenu est estimé. Ces résultats sont comparés aux résultats mesurés et les changements graduels de la propriété du matériau sont calculés. Les propriétés mises à jour du matériau sont utilisées pour recalculer le champ.

Le document CN 111067524 A (UNIV TIANJIN) (2020-04-28) divulgue un procédé d'estimation des propriétés diélectriques moyennes de l'imagerie mammaire par micro-ondes. Le procédé comprend les étapes suivantes : étirer chaque image source IRM mammaire, discrétiser chaque tissu mammaire, ajouter une couche de peau en prenant comme référence le contour de la poitrine, et établir un modèle mammaire tridimensionnel par un traitement d'interpolation ; définir une position de tumeur et un rayon de tumeur dans un modèle tridimensionnel du sein, disposer un réseau d'antennes à la surface de la peau, effectuer un remplacement par une source ponctuelle, permettre à chaque antenne d'émettre des signaux de manière séquentielle, permettre aux autres antennes de recevoir des signaux, et soumettre tous les signaux reçus à un traitement d'imagerie par un algorithme confocal ; d'obtenir une constante diélectrique moyenne correspondante du sein.

Le document US 2005/107692 A1 (LI JIAN [US] ET AL) (2005-05-19) divulgue un système d'examen d'un tissu biologique, qui consiste à irradier une zone tissulaire avec une pluralité d'impulsions de rayonnement hyperfréquence. Les impulsions hyperfréquence sont balayées sur toute une plage de fréquences micro-ondes. En réponse aux impulsions hyperfréquence balayées, la zone tissulaire émet une pluralité de signaux thermoacoustiques. Une image de la zone tissulaire est formée ensuite à partir de la pluralité de signaux thermoacoustiques.

Le document US 2019/175095 A1 (BORE CHRIS [GB]) (2019-06-13) divulgue un système d'imagerie médicale qui comprend un réseau d'antennes hyperfréquences : des antennes d'émission et de réception espacées les unes des autres. Le réseau d'antennes à micro-ondes a plusieurs configurations définissant les positions des antennes par rapport à la partie de corps. Le système d'imagerie médicale comprend en outre un actionneur conçu pour déplacer le réseau d'antennes à micro-ondes entre les configurations et un processeur configuré pour obtenir un ensemble de données pour les signaux micro-ondes produits dans chacune des configurations du réseau d'antennes à micro-ondes et pour générer une sortie indicative de la structure interne de la partie corporelle à partir d'une concaténation des ensembles de données.

Le document "Super-resolution radar imaging for breast cancer detection with microwaves: the integrated information selection criteria",par FASOULA A. ET. AL.: 2019 41ST ANNUAL INTERNATIONAL CONFERENCE OF THE IEEE ENGINEERING IN MEDICINE AND BIOLOGY SOCIETY (EMBC), IEEE, 23 juillet 2019 (2019-07-23), pages 1868-1874, divulgue un schéma de prétraitement des données sur les modules de sélection de fréquence et de filtrage spatial intégrés à un algorithme TR-MUSIC (Time-Reversal Multiple Signal Classification) pour l'imagerie mammaire par micro-ondes. Un nouveau schéma de sélection de fréquence est proposé, de sorte que l'algorithme d'imagerie TR-MUSIC soit appliqué aux fréquences discrètes qui transmettent la plupart des informations utiles provenant de l'intérieur du sein. Un critère de sélection de sous-images, basé sur l'équilibre de l'intensité de l'image entre l'extérieur et l'intérieur du sein, permet de renforcer encore la robustesse du système d'imagerie contre le couplage résiduel ou d'autres sources d'interférence. Enfin, une approche de filtrage spatial limitant la contribution de chaque secteur (sous-ensemble) de capteurs à son voisinage (c'est-à-dire la zone la mieux éclairée par l'ensemble d'antennes donné) est présentée.

EXPOSE DE L'INVENTION

L'invention permet d'améliorer la qualité des images radar pour l'analyse de tissus ou d'organes humains. En particulier, l'invention permet de détecter dans les images radar une ou plusieurs région(s) d'intérêt(s) qui correspondent à des objets physiques susceptibles d'être des lésions.

A cet effet, l'invention propose, selon un premier aspect, un procédé de traitement d'images médicales selon la revendication 1.

L'invention est avantageusement complétée par les caractéristiques suivantes, prises seules ou en une quelconque de leur combinaison techniquement possible :

- le traitement morphologique consiste à déterminer la solidité d'une ou plusieurs régions de pixels de l'image, une région d'intérêt étant identifiée si la solidité associée est supérieure à un seuil.
- on considère au moins N=2 ensembles, de préférence au moins N=3 ensembles de Ai > 1 valeurs, $1 \leq i \leq N,$ de paramètre caractéristique (pcfib) du milieu traversé par les signaux.
- les ensembles se recoupent totalement ou partiellement en termes de plages de variation et/ou en termes de valeurs.
- l'évaluation de la persistance consiste à déterminer un pourcentage de présence d'une région d'intérêt sur les images morphologiques, une région d'intérêt étant validée pour un pourcentage supérieur à un seuil.
- le réseau de sondes comprend K > 1 sondes disposées autour de la zone à imager, ledit réseau étant mobile selon des positions verticales autour de la zone à imager, chaque configuration étant un secteur angulaire de sondes chacun composé de M > 1 sondes avec M < K, chaque configuration étant décalée angulairement d'au moins une sonde par rapport à une autre configuration ; pour chaque secteur, chacune des sondes est en émission à tour de rôle de manière à produire les signaux acquis pour la configuration, une image radar élémentaire étant déterminée pour chaque secteur angulaire par ensemble de valeurs de paramètre caractéristique.

L'invention propose selon un deuxième aspect, un produit programme d'ordinateur comprenant des instructions de code de programme pour l'exécution des étapes du procédé selon le premier aspect de l'invention, lorsque ce procédé est exécuté par au moins un processeur.

L'invention propose selon un troisième aspect un dispositif d'imagerie médicale comprenant une unité de traitement configurée pour mettre en œuvre un procédé selon le premier aspect de l'invention.

La combinaison de plusieurs configurations et l'utilisation comme hypothèses de plusieurs valeurs caractéristiques du milieu traversé permet de gérer l'hétérogénéité des propriétés diélectriques non connues a priori de la zone à imager.

En outre, la persistance d'une région d'intérêt identifiée morphologiquement pour au moins B > 1 ensembles parmi N ensembles (avec $B/N \leq 1$) implique une validation de la région d'intérêt c'est-à-dire une forte probabilité qu'elle corresponde à un objet physique/lésion présente dans la zone imagée versus artefact.

De manière complémentaire, le critère de solidité utilisé est un descripteur de forme qui permet une identification morphologique avantageuse des régions d'intérêts.

PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :

- la figure 1 illustre schématiquement un système d'imagerie médicale à micro-ondes conforme à un mode de réalisation de l'invention ;
- la figure 2 illustre des étapes d'un procédé de traitement morphologique d'images radar micro-ondes selon l'invention ;
- la figure 3 illustre une image radar micro-ondes obtenue au cours d'un procédé selon l'invention ;
- la figure 4a, la figure 4b, la figure 4c, la figure 4d, la figure 4e illustrent des images morphologiques du sein d'une patiente, obtenues au moyen d'un procédé de traitement morphologique d'images radar micro-ondes selon l'invention.
- la figure 5a, la figure 5b, la figure 5c, la figure 5d illustrent des images morphologiques du sein d'une patiente, obtenues au moyen d'un procédé de traitement morphologique d'images radar micro-ondes selon l'invention.

Sur l'ensemble des figures les éléments similaires portent des références identiques.

DESCRIPTION DETAILLEE

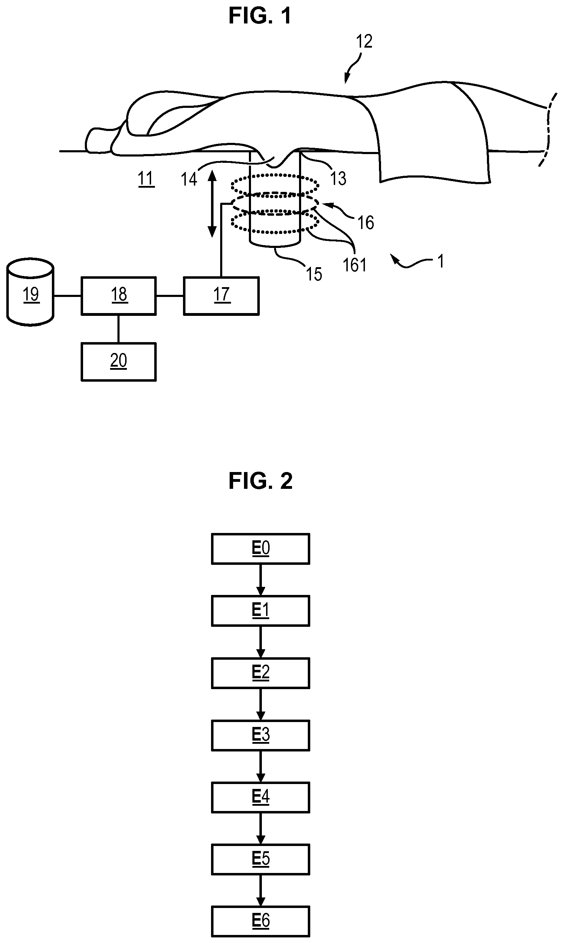

La **figure 1** illustre un dispositif 1 d'imagerie médicale à micro-ondes comprenant une table d'examen 11 sur laquelle une patiente 12 est allongée. En particulier, la patiente 12 est allongée en position couchée sur le ventre. La table d'examen 11 comprend une ouverture 13 de préférence circulaire permettant l'immersion du sein 14 de la patiente dans une cuve 15 remplie d'un liquide de transition biocompatible dont les propriétés diélectriques sont optimisées pour améliorer la transmission des ondes électromagnétiques à l'intérieur du sein.

Un réseau 16 de sondes 161 d'émission/réception d'ondes électromagnétiques dans le domaine des micro-ondes (ci-après schématisées par des tirets) est disposé autour de la cuve 15 et permet en émission d'illuminer le milieu observé et en réception de recevoir les signaux réfléchis depuis la scène à imager. Les sondes 161 sont avantageusement régulièrement réparties autour de la cuve et préférentiellement selon un anneau entourant la cuve comme cela est illustré sur la **figure 1.** Avantageusement, les sondes sont configurées pour émettre des signaux dans la bande de fréquences 0,5 - 6 GHz.

Plus généralement, le système d'imagerie fonctionne de manière multistatique et permet d'illuminer le milieu à imager en exploitant plusieurs sondes en émission et plusieurs sondes en réception et selon différentes configurations autour du milieu à imager. Les sondes peuvent, de manière complémentaire, être configurées pour émettre et recevoir simultanément.

A chaque acquisition multistatique, tout ou partie du milieu à imager est éclairé successivement par des sondes présélectionnées qui fonctionnent en émission. Les sondes en émission du réseau et leur nombre sont choisis en fonction de la zone du sein à imager. Pour chaque sonde qui émet, le signal est reçu par des sondes présélectionnées qui fonctionnent en réception. Les sondes en réception du réseau et leur nombre sont choisis en fonction de la zone du sein à imager. On considère alors que chaque acquisition multistatique correspond à une série d'émissions/réceptions de signaux par des sondes selon une configuration déterminée.

On entend ainsi par configuration la définition d'un ensemble de sondes d'émission et la définition d'un ensemble de sondes de réception permettant d'effectuer une acquisition multistatique de tout ou partie du sein, ces sondes étant disposées d'une certaine manière dans l'espace autour du sein.

Pour passer d'une configuration à une autre et pour piloter les différentes acquisitions multistatiques, le système comprend une unité 17 de pilotage du réseau de sondes qui est connectée à une unité 18 de contrôle et de traitement (par exemple un processeur et/ou un calculateur). Une telle unité 18 de contrôle et de traitement est configurée pour piloter le réseau, procéder aux acquisitions, assurer le stockage des données acquises, effectuer des traitements d'imagerie radar et mettre en œuvre un procédé de traitement morphologique d'images qui sera décrit ci-après. Une unité 19 de stockage permet de stocker l'ensemble des données multistatiques acquises et un certain nombre de données qui peuvent être utilisées pour les étapes du traitement d'imagerie ou produites par le traitement d'imagerie. En outre, une unité 20 d'affichage permet d'afficher et de visualiser des images obtenues. L'unité 18 de contrôle et de traitement, l'unité 19 de stockage et l'unité 20 d'affichage peuvent être intégrées directement dans l'appareil d'imagerie ou bien déportées physiquement. Les traitements d'imagerie peuvent être réalisés a posteriori (off-line).

Comme on l'aura compris, pour imager l'ensemble du sein plusieurs configurations successives de sondes d'émission et de sondes de réception sont définies. Ces configurations de sondes d'émission et de sondes de réception couvrent des zones différentes du sein à imager et sont choisies de façon à englober au final l'ensemble du sein à imager.

Pour chaque configuration, les acquisitions multistatiques des coefficients de transmission entre les sondes d'émission et les sondes de réception permettent, après traitement d'imagerie radar, d'obtenir une image radar élémentaire.

L'ensemble des images élémentaires obtenues permettent de reconstruire une image radar 2D ou 3D de la zone imagée, ici le sein. Pour le traitement radar des signaux multistatiques d'émission/réception permettant la reconstruction des images radar 2D ou 3D, on pourra par exemple se référer aux publications suivantes :

- A.J. Devaney, Time reversal imaging of obscured targets from multistatic data, IEEE Trans. Antennas Propag. (2005). doi:10.1109/TAP.2005.846723;
- Marengo, E.A.; Gruber, F.K.; Simonetti, F. Time-reversal MUSIC imaging of extended targets. IEEE Trans. Image Process. 2007, 16, 1967-1984. doi:10.1109/TIP.2007.899193;
- Hossain, M.D.; Mohan, A.S. Cancer Detection in Highly Dense Breasts Using Coherently Focused Time-Reversal Microwave Imaging. IEEE Trans. Comput. Imaging 2017, 3, 928-939. doi:10.1109/TCI.2017.2737947;
- A. Fasoula, B.M. Moloney, L. Duchesne, J.D.G. Cano, B.L. Oliveira, J. Bernard, M.J. Kerin, Super-resolution radar imaging for breast cancer detection with microwaves: the integrated information selection criteria, in: 41st Annu. Int. Conf. IEEE Eng. Med. Biol. Soc., 2019.

Les images radar 2D ou 3D obtenues sont avantageusement exploitées dans le cadre d'un procédé de traitement qui va être décrit ci-après.

Comme mentionné, en introduction, la détermination de chaque image radar élémentaire nécessite en théorie de connaitre a priori le milieu diélectrique du sein (c'est-à-dire du milieu traversé) le long du chemin parcouru entre chaque sonde d'émission et chaque sonde de réception. Or ceci n'est pas accessible.

Comme il va être décrit, l'invention met en œuvre un paramètre pcfib qui correspond à une hypothèse sur la constitution moyenne du milieu traversé par l'onde électromagnétique dans le sein (ou de manière plus générale, la zone imagée) en termes de permittivité diélectrique. Ce paramètre pcfib correspond à un pourcentage de mélange de tissus fibro-glandulaires et de tissus adipeux du sein. Par exemple pcfib = 30% correspond à un milieu contenant 30% de tissus fibro-glandulaires et 70% de tissus adipeux. Les propriétés diélectriques du tissu mammaire sont alors définies comme une moyenne pondérée (pondération par pcfib) des propriétés diélectriques des tissus fibro-glandulaires et des tissus adipeux. Pour des exemples de valeurs des permittivités diélectriques des tissus fibro-glandulaires et des tissus adipeux du sein, on pourra par exemple se référer aux publications suivantes :

- T. Sugitani, S.I. Kubota, S.I. Kuroki, K. Sogo, K. Arihiro, M. Okada, T. Kadoya, M. Hide, M. Oda, T. Kikkawa, Complex permittivities of breast tumor tissues obtained from cancer surgeries, Appl. Phys. Lett. (2014). doi:10.1063/1.4885087;
- M. Lazebnik, L. McCartney, D. Popovic, C.B. Watkins, M.J. Lindstrom, J. Harter, S. Sewall, A. Magliocco, J.H. Booske, M. Okoniewski, S.C. Hagness, A large-scale study of the ultrawideband microwave dielectric properties of normal breast tissue obtained from reduction surgeries, Phys. Med. Biol. (2007). doi:10.1088/0031-9155/52/10/001.

On décrit ci-après un procédé de traitement morphologique d'images radar micro-ondes en relation avec la **figure 2.**

Dans un premier temps on définit P > 1 configuration(s) du réseau de sondes (étape E0), de manière à pouvoir englober l'ensemble du sein à imager et reconstruire, par la suite, une image radar 3D du sein.

Puis pour chaque configuration, une acquisition multistatique des coefficients de transmission mesurés entre les sondes émettrices et les sondes réceptrices est réalisée (étape E1). On dispose alors de plusieurs acquisitions multistatiques (P > 1 acquisitions multistatiques).

Ensuite, les signaux acquis pour chaque configuration sont traités afin d'obtenir des images radar élémentaires micro-ondes pour chacune des configurations (étape E2).

En particulier, pour traiter ces signaux on considère plusieurs ensembles (N > 1 ensembles) de $A_i$ valeurs ($A_i$ > 1 valeurs, avec $1 \leq i \leq N$) du paramètre pcfib. On a alors $\sum_{i=1}^{N} A_i$ valeurs de paramètres pcfib par configuration. Ainsi, on obtient à partir des signaux de chaque acquisition multistatique $\sum_{i=1}^{N} A_i$ images radar élémentaires micro-ondes chacune ayant été obtenue pour une valeur du paramètre pcfib. L'idée ici est d'obtenir des images élémentaires selon différentes hypothèses sur le milieu traversé par les ondes électromagnétiques.

De manière avantageuse, les ensembles de valeurs du paramètre pcfib se recoupent totalement ou partiellement en termes de variation et/ou en termes de valeurs.

Par exemple, on peut avoir un ensemble comprenant les valeurs 10%, 20% et un autre ensemble comprenant les valeurs 5%, 15% 25%. Dans cet exemple on a un ensemble dont les valeurs varient entre 10% et 20% et un autre ensemble dont les valeurs varient entre 5% et 25%. Ces deux ensembles ont donc une plage de variation commune entre 10% et 20%.

Dans un autre exemple, on peut avoir un ensemble comprenant les valeurs 10%, 20% et un autre ensemble comprenant les valeurs 20%, 25%, 30%. Dans cet exemple les ensembles possèdent une valeur en commun, 20%.

Dans encore un autre exemple, on peut avoir un ensemble comprenant les valeurs 10%, 20%, 25% et un autre ensemble comprenant les valeurs 5% 10% 30%. Dans cet exemple, ces deux ensembles ont une plage de variation commune entre 10% et 25% et une valeur commune 10%.

On considère au moins deux ensembles de valeurs du paramètre pcfib dont un ensemble pouvant avoir une plage de variation des valeurs du paramètre pcfib plus large que la plage de variation de l'autre ensemble. Ici les termes large et étroit sont des termes relatifs qui s'entendent en comparant les plages de variation. L'idée ici est d'avoir des recoupements entre les ensembles de valeurs.

Le choix des plages de variation du paramètre pcfib pour les différents ensembles est effectué en lien avec la variabilité existante en termes de compositions et de densité des seins.

De manière avantageuse, des plages de variation larges conduisent à des images comprenant une représentation plus complète de la région d'intérêt et les plages de variation étroites conduisent potentiellement à des représentations partielles des lésions détectables.

Par exemple, dans le cadre de l'imagerie du sein on peut choisir N=5 ensembles de variation :

- trois ensembles avec des plages de variation étroites :

  ○ entre 10% et 20%, le paramètre pcfib prenant par exemple les valeurs suivantes dans cette plage :10%, 15%, 20%
  ○ entre 30% et 40%, le paramètre pcfib prenant par exemple les valeurs suivantes dans cette plage : 30%, 35%, 40%

∘ entre 50% et 60%, le paramètre pcfib prenant par exemple les valeurs suivantes dans cette plage : 50%, 55%, 60%

- deux ensembles avec des plages de variation larges :

∘ entre 20% et 50 %, le paramètre pcfib prenant par exemple les valeurs suivantes dans cette plage : 20%, 25%, 30%, 35%, 40%, 45%, 50%
∘ entre 10% et 60%, le paramètre pcfib prenant par exemple les valeurs suivantes dans cette plage : 10%, 15%, 20%, 25%, 30%, 35%, 40%, 45%, 50%, 55%, 60%.

Pour chaque configuration, une image radar élémentaire micro-ondes est sélectionnée pour chaque ensemble (étape E3) et correspond à une des valeurs du paramètre (pcfib) de l'ensemble ; pour chaque configuration, une image élémentaire par ensemble est donc sélectionnée. Une telle image sélectionnée est susceptible de contenir une région d'intérêt correspondant à un objet physique potentiellement une lésion : une telle image sélectionnée est susceptible de contenir une région d'intérêt correspondant à un objet physique potentiellement une lésion. Dans l'exemple précédent, on a alors cinq images élémentaires par configuration (une image élémentaire par ensemble) qui vont être utilisées pour la reconstruction.

Une telle sélection consiste notamment à utiliser au moins un des critères de focalisation d'image (en anglais, image focusing metrics) tels que, par exemple, les critères décrits dans les documents suivants (une combinaison de plusieurs critères peut être utilisée) :

- S. Pertuz, D. Puig, M.A. Garcia, Analysis of focus measure operators for shape-from-focus, Pattern Recognit. (2013). Doi :10.1016/j.patcog.2012.11.011.
- O'loughlin, D.; Krewer, F.; Glavin, M.; Jones, E.; O'halloran, M. Focal quality metrics for the objective evaluation of confocal microwave images. Int. J. Microw. Wirel. Technol. 2017, 9, 1365-1372. Doi :10.1017/S1759078717000642.

Chaque image sélectionnée est susceptible de contenir une ou plusieurs régions d'intérêt, sélection effectuée selon au moins un critère de focalisation d'image. En effet, la sélection se fait au moyen d'un ou plusieurs critères de focalisation. Il s'agit par exemple pour un critère choisi de sélectionner l'image qui donne par exemple, la valeur de métrique minimale, parmi toutes les autres pour ce critère.

On note que d'une configuration à l'autre, la sélection de l'image élémentaire pour un même ensemble donné peut avoir été effectuée avec des valeurs différentes du paramètre pcfib appartenant à cet ensemble.

A partir des images élémentaires obtenues pour les différentes configurations, une image radar 2D ou 3D de la zone imagée est reconstruite pour chacun des ensembles (étape E4). Ainsi, on dispose d'une image radar reconstruite par ensemble de valeurs de pcfib.

Sur chaque image radar micro-ondes ainsi reconstruite de la zone imagée est appliqué un traitement morphologique de manière à détecter des régions d'intérêt, si existantes (étape E5). Le résultat obtenu est une image dite morphologique micro-ondes contenant aucune, une ou plusieurs régions d'intérêt identifiée(s). Un tel traitement morphologique consiste notamment à identifier les objets connectés dans l'image en utilisant un procédé de seuillage et à retenir comme régions d'intérêt les objets connectés qui correspondent à un ensemble de caractéristiques morphologiques, notamment, la taille volumétrique de l'objet connecté, le niveau de solidité de l'objet connecté, le niveau d'intensité à l'intérieur de l'objet connecté, le niveau de contraste entre l'intensité à l'intérieur de l'objet connecté et l'intensité à l'intérieur d'autres objets connectés potentiellement identifiés dans la même image. A ce stade, on dispose alors de plusieurs images morphologiques du sein, chaque image morphologique étant obtenue par ensemble de valeurs de pcfib ; chaque image morphologique contenant aucune, une ou plusieurs régions d'intérêt identifiée(s).

De manière préférée, le traitement morphologique est basé sur le critère de solidité. La solidité est calculée comme le rapport entre le volume de l'objet et le volume de l'enveloppe convexe de l'objet. En général, plus la solidité d'une région d'intérêt augmente, plus cette région d'intérêt sera « remplie » (région d'intérêt sans trous), plus elle possédera un contour bien défini et convexe et donc plus elle aura une probabilité importante de correspondre à une masse. Dans le cas de l'imagerie du sein, ce critère de solidité peut être destiné à soutenir la différenciation entre une masse mammaire et une asymétrie focale («îlot» de tissu mammaire normal, sans bordure extérieure convexe définie). Cette notion de contour bien défini et convexe d'une masse mammaire solide est, par exemple, explicitée dans les publications suivantes :

- T.F. de Brito Silva, A.C. de Paiva, A.C. Silva, G. Braz Júnior, J.D.S. de Almeida, Classification of breast masses in mammograms using geometric and topological feature maps and shape distribution, Res. Biomed. Eng. (2020). doi:10.1007/s42600-020-00063-x ;
- N. Safdarian, M. Hedyezadeh, Detection and Classification of Breast Cancer in Mammography Images Using Pattern Recognition Methods, Multidiscip. Cancer Investig. (2019). doi:10.30699/acadpub.mci.3.4.13.

En pratique la solidité d'une région d'intérêt doit dépasser un niveau donné pour que cette région d'intérêt puisse être identifiée dans une image morphologique d'un ensemble donné.

Puis, la persistance de chaque région d'intérêt préalablement identifiée est évaluée sur les différentes images morphologiques. L'objectif est de valider morphologiquement les régions d'intérêt qui sont persistantes pour plusieurs hypothèses sur le milieu traversé par les ondes électromagnétiques (étape E6). On entend par évaluation de la persistance le fait d'avoir sur plusieurs images morphologiques la présence d'une région d'intérêt localisée en 3D dans une même zone. Ici on va évaluer si des régions d'intérêt identifiées par le traitement morphologique se trouvent sur plusieurs images dans une même zone. Une telle évaluation consiste notamment à utiliser des critères comme, par exemple, le regroupement spatial (en anglais, spatial clustering criterion) pour associer entre elles les régions d'intérêt détectées.

La persistance permet donc de valider morphologiquement la région d'intérêt, c'est-à-dire son association à un objet physique si la région d'intérêt est présente sur une proportion déterminée du nombre d'images morphologiques.

Comme indiqué, de manière avantageuse, on considère au moins deux ensembles de valeurs de pcfib et de préférence au moins trois ensembles de valeurs de pcfib. Ceci est important pour mettre en œuvre l'étape d'évaluation de la persistance. En effet, une région d'intérêt sera considérée comme valide si elle est persistante sur plusieurs images morphologiques. Dans le cas de deux ensembles, pour que la région d'intérêt soit valide il faut qu'elle soit présente sur les deux images. Dans le cas de trois ensembles, pour que la région d'intérêt soit valide il faut qu'elle soit présente sur deux images sur trois ou trois images sur trois.

De manière générale, on va considérer qu'une région d'intérêt est persistante si elle est présente sur au moins un pourcentage d'images morphologiques qui doit être définit en fonction du type de région recherché.

Ces régions d'intérêt ainsi validées au moyen de la persistance ont alors une forte probabilité de correspondre à une lésion ou une tumeur réelle, plutôt qu'à un artefact par exemple. Cette persistance peut être associée à un degré de confiance au niveau de la détection.

De manière complémentaire, on considère que le système d'imagerie comprend un réseau circulaire horizontal de K > 1 sondes de mesure placées autour d'un cylindre en matériau diélectrique. Le réseau circulaire a la possibilité de se déplacer suivant l'axe vertical. L'organe sous test (sein) est placé dans le cylindre et est donc entouré par ce réseau. Un milieu de transition diélectrique contenu dans le cylindre où le sein est placé permet d'optimiser la pénétration des ondes électromagnétiques émises par les sondes à l'intérieur du sein. Les positions verticales du réseau sont prédéfinies, avec des intervalles de distance constants ou variables entre chaque position, et couvrent l'étendue verticale du sein.

Des mesures multistatiques sont réalisées pour chacune des positions verticales du réseau de sondes.

Comme exemple de mise en œuvre de l'étape d'acquisition, L > 1 secteurs angulaires composés chacun de M > 1 sondes du réseau de sondes (M < K) sont considérés. Par exemple L=18 secteurs angulaires de M=6 sondes avec, dans ce cas, chaque secteur décalé angulairement relativement l'un par rapport à l'autre de R=1 sonde le long du pourtour d'un réseau de K=18 sondes.

Pour chacun des secteurs angulaires de M=6 sondes, chacune des sondes est en émission à tour de rôle, et pour chaque sonde émettrice, les autres sondes du secteur angulaire reçoivent successivement le signal correspondant aux échos provenant des obstacles rencontrés, notamment dans le sein. L'ensemble des coefficients de transmission mesurés entre les sondes émettrices et les sondes réceptrices du secteur angulaire considéré forment une acquisition multistatique. Cette acquisition multistatique est répétée pour l'ensemble des L=18 secteurs angulaires de M=6 sondes. Puis le réseau vertical est déplacé d'un intervalle suivant l'axe vertical et les mesures multistatiques sont répétées de nouveau pour les différents secteurs angulaires.

Une configuration du réseau correspond alors à un secteur angulaire à une position verticale donnée. Les acquisitions par secteur angulaire permettent de faire le tour du sein en faisant plusieurs hypothèses sur les valeurs de pcfib à chaque secteur angulaire à chaque position verticale. Ceci permet de tenir compte de manière optimale de la structure variable et hétérogène du sein en termes de propriétés diélectriques qui peuvent changer selon les différentes positions d'observation et de révéler la réponse angulaire non uniforme des lésions mammaires.

Ainsi, on profite de la sectorisation de la zone à imager en combinaison avec les différentes hypothèses sur le paramètre pcfib pour améliorer la détection des régions d'intérêt.

*Types d'images obtenues avec l'invention*

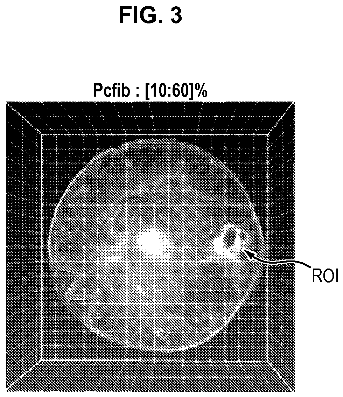

La **figure 3** illustre une image radar micro-onde reconstruite pour un ensemble de valeurs de pcfib telles que $10\% \leq pcfib \leq 60\%$. Cette image radar est représentée dans la vue coronale du sein. Sur cette image radar plusieurs régions de pixels ressortent en termes d'intensité.

L'objectif du traitement morphologique est de traiter ce type d'images radar pour identifier des régions d'intérêts qui pourraient correspondre à des zones suspectes.

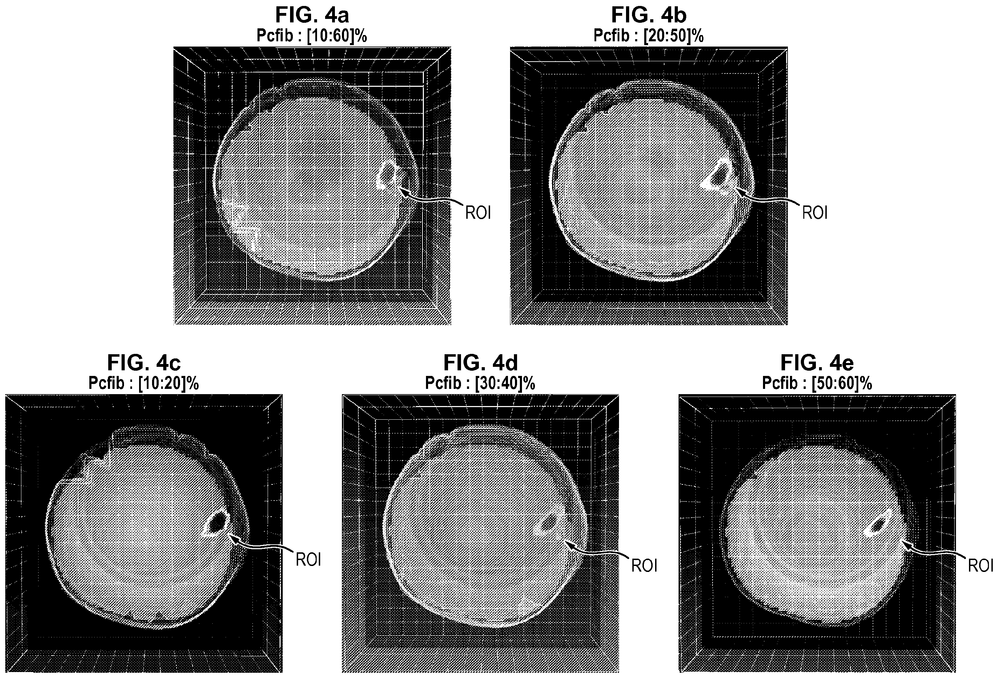

Les **figures 4a, 4b, 4c, 4d** et **4e** illustrent plusieurs images morphologiques du sein d'une patiente, obtenues après traitement morphologique des images radar micro-ondes reconstruites pour cinq ensembles de valeurs du paramètre pcfib. Ces images morphologiques sont représentées dans la vue coronale du sein. Dans cet exemple, les images morphologiques correspondent à cinq ensembles de valeurs pour le paramètre pcfib :

- **figure 4a** : 10% ≤ pcfib ≤ 60%
- **figure 4b** : 20% ≤ pcfib ≤ 50%
- **figure 4c** : 10% ≤ pcfib ≤ 20%
- **figure 4d** : 30% ≤ pcfib ≤ 40%
- **figure 4e** : 50% ≤ pcfib ≤ 60%

Au regard de l'image radar de la **figure 3,** le traitement morphologique a permis de n'identifier qu'une seule région d'intérêt. Cette région d'intérêt est persistante sur les cinq images morphologiques et est donc validée.

On remarque que sur chaque image morphologique la région d'intérêt identifiée possède des contours différents, ce qui confirme que la signature radar micro-ondes de l'objet détecté varie en fonction des ensembles des valeurs pcfib considérés.

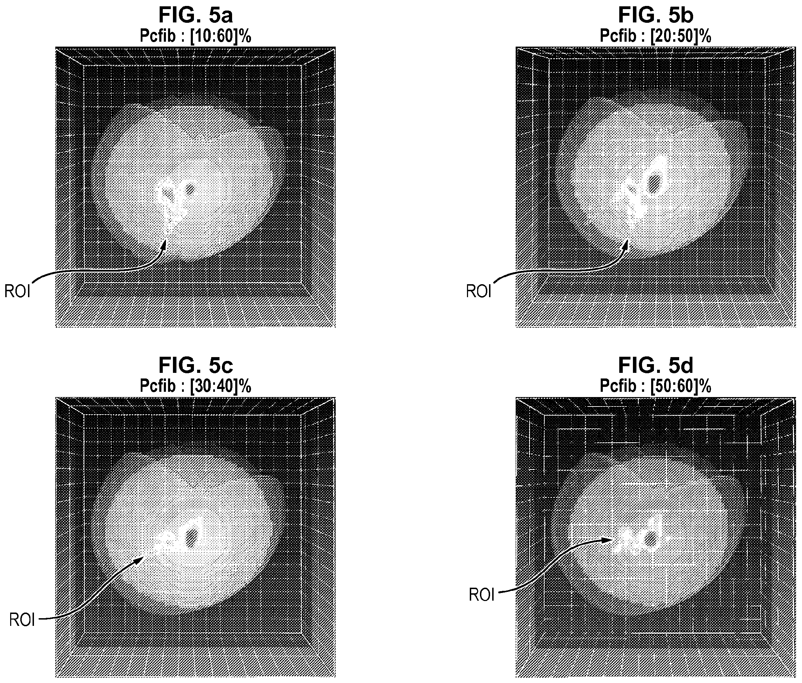

Les **figures 5a, 5b, 5c** et **5d** illustrent des images morphologiques du sein d'une autre patiente obtenues après traitement morphologique des images radar micro-ondes reconstruites aussi pour cinq ensembles de valeurs du paramètre pcfib. Ces cinq ensembles de valeurs sont identiques à ceux considérés dans le cas des figures précédentes (la patiente précédente). Dans cet exemple, le traitement morphologique a permis d'identifier une seule région d'intérêt qui était persistante sur quatre des cinq images morphologiques. Cette région d'intérêt est donc validée. La cinquième image morphologique correspondant à l'ensemble des valeurs 10% ≤ pcfib ≤ 20% n'est pas représentée car aucune région d'intérêt persistante n'y a été identifiée. A la différence de l'exemple précédent, la région d'intérêt identifiée se présente sous une forme de constellation mais qui reste un même et seul objet connecté d'après les traitements précédemment appliqués. Cela illustre que la signature radar micro-ondes de l'objet détecté varie très significativement en fonction des ensembles des valeurs pcfib considérés. La région d'intérêt correspond à une lésion de forme distribuée, très irrégulière et d'une texture hautement hétérogène.

## Revendications

1. Procédé de traitement d'images médicales de tissus humains d'une zone du corps d'un patient et en particulier du sein au moyen d'un dispositif (1) d'imagerie médicale comprenant un réseau de sondes émettrices/réceptrices d'ondes électromagnétiques dans le domaine des micro-ondes constitué de K > 1 sondes espacées les unes des autres, le réseau comprenant P > 1 configurations différentes définissant des sondes émettrices et des sondes réceptrices pour une ou plusieurs position(s) autour de la zone, dans lesquelles les sondes émettrices sont configurées pour émettre des signaux micro-ondes de manière à éclairer une zone du corps et les sondes réceptrices sont configurées pour recevoir des signaux micro-ondes après diffusion et réflexion dans la zone, les sondes pouvant, de manière complémentaire, être configurées pour émettre et recevoir simultanément, le procédé comprenant les étapes suivantes mises en oeuvre dans une unité de traitement du dispositif d'imagerie médicale :

- acquisition (E1) des signaux produits dans P > 1 configurations du réseau d'antennes ;
- le procédé comprenant pour chaque configuration :

 - traitement (E2) des signaux acquis pour N > 1 ensembles de Ai > 1 valeurs, $1 \leq i \leq N,$ d'un paramètre caractéristique (pcfib) des propriétés diélectriques des tissus humains traversés par les signaux, de manière à obtenir $\sum_{i=1}^{N} Ai$ images radar élémentaires micro-ondes ;
 - sélection (E3) dans chacun des N ensembles suivant au moins un critère de focalisation d'image, d'une image radar élémentaire micro-ondes, chaque image élémentaire sélectionnée correspondant à une des valeurs du paramètre (pcfib) de l'ensemble ; une image élémentaire par ensemble étant sélectionnée pour une configuration ;

- le procédé comprenant pour chaque ensemble :

 - reconstruction (E4) à partir des images radar élémentaires sélectionnées de chacune des configurations d'une image radar de la zone du corps d'un patient de manière à reconstruire une image radar 3D par ensemble ;

le procédé étant **caractérisé en ce qu'**il comprend en outre pour chaque ensemble:

- traitement morphologique (E5) de chaque image radar reconstruite de manière à obtenir une image morphologique sur laquelle est identifiée une ou plusieurs régions d'intérêt susceptible(s) d'être une lésion, si existante(s),
- évaluation (E6) de la persistance de chaque région d'intérêt sur les différentes images morphologiques obtenues de manière à valider morphologiquement la région d'intérêt, l'évaluation de la persistance consistant à évaluer si des régions d'intérêts se trouvent sur plusieurs images morphologiques dans une même zone.

2. Procédé selon la revendication 1, dans lequel le traitement morphologique (E5) consiste à déterminer la solidité d'une ou plusieurs régions de pixels de l'image, une région d'intérêt étant identifiée si la solidité associée est supérieure à un seuil.

3. Procédé de traitement selon l'une des revendications précédentes, dans lequel on considère au moins N=2 ensembles, de préférence au moins N=3 ensembles de Ai > 1 valeurs, $1 \leq i \leq N$, de paramètre caractéristique (pcfib) du milieu traversé par les signaux.

4. Procédé selon la revendication 3, dans lequel les ensembles se recoupent totalement ou partiellement en termes de plages de variation et/ou en termes de valeurs.

5. Procédé selon l'une des revendications précédentes, dans lequel l'évaluation de la persistance consiste à déterminer un pourcentage de présence d'une région d'intérêt sur les images morphologiques, une région d'intérêt étant validée pour un pourcentage supérieur à un seuil.

6. Procédé selon la revendication précédente, dans lequel le réseau de sondes comprend K > 1 sondes disposées autour de la zone à imager, ledit réseau étant mobile selon des positions verticales autour de la zone à imager, chaque configuration étant un secteur angulaire de sondes chacun composé de M > 1 sondes avec M < K, chaque configuration étant décalée angulairement d'au moins une sonde par rapport à une autre configuration ; pour chaque secteur angulaire, chacune des sondes est en émission à tour de rôle de manière à produire les signaux acquis pour la configuration, une image radar élémentaire étant déterminée pour chaque secteur angulaire par ensemble de valeurs de paramètre caractéristique (pcfib).

7. Produit programme d'ordinateur comprenant des instructions de code de programme pour l'exécution des étapes du procédé selon l'une des revendications 1 à 6, lorsque ce procédé est exécuté par au moins un processeur.

8. Dispositif d'imagerie médicale comprenant une unité de traitement configurée pour mettre en œuvre un procédé selon l'une des revendications 1 à 6.

**Patentansprüche**

1. Verfahren zur Verarbeitung medizinischer Bilder von menschlichem Gewebe eines Körperbereichs eines Patienten, und insbesondere der Brust, mittels einer medizinischen Bildgebungsvorrichtung (1), die ein Netzwerk von Sende-/Empfangssonden elektromagnetischer Wellen im Mikrowellenbereich umfasst, das aus K > 1 voneinander beabstandeten Sonden besteht, wobei das Netzwerk P > 1 verschiedene Konfigurationen umfasst, die Sendersonden und Empfangssonden für eine oder mehrere Position(en) um den Bereich herum definieren, wobei die Sendersonden dazu ausgelegt sind, Mikrowellensignale derart zu senden, dass ein Bereich des Körpers beleuchtet wird und die Empfangssonden dazu ausgelegt sind, Mikrowellensignale nach Streuung und Reflexion in dem Bereich zu empfangen, wobei die Sonden zusätzlich dazu ausgelegt sein können, gleichzeitig zu senden und zu empfangen, wobei das Verfahren die folgenden Schritte umfasst, die in einer Verarbeitungseinheit der medizinischen Bildgebungsvorrichtung durchgeführt werden:

- Erfassen (E1) der Signale, die in P > 1 Konfigurationen des Antennennetzwerks erzeugt werden;
- wobei das Verfahren für jede Konfiguration umfasst:

- Verarbeiten (E2) der erfassten Signale für N > 1 Gruppen von Ai > 1 Werten, $1 \leq i \leq N$, eines charakteristischen Parameters (pcfib) der dielektrischen Eigenschaften der von den Signalen durchquerten menschlichen Gewebe, so dass $\sum_{i=1}^{N} Ai$ elementare Mikrowellen-Radarbilder erhalten werden;
- Auswählen (E3) in jeder der N Gruppen nach mindestens einem Bildfokussierungskriterium eines elementaren Mikrowellen-Radarbildes, wobei jedes ausgewählte elementare Bild einem der Werte des Parameters (pcfib) der Gruppe entspricht; wobei ein elementares Bild pro Gruppe für eine Konfiguration ausgewählt wird;
- wobei das Verfahren für jede Gruppe umfasst:

- Wiederherstellen (E4), aus den ausgewählten elementaren Radarbildern jeder der Konfigurationen, eines Radarbildes des Körperbereichs eines Patienten derart, dass ein 3D-Radarbild pro Gruppe wiederhergestellt wird;
- morphologisches Verarbeiten (E5) jedes wiederhergestellten Radarbildes derart, dass ein morphologisches Bild erhalten wird, auf dem ein oder mehrere Bereiche von Interesse identifiziert werden, die eine Läsion darstellen könnten, sofern vorhanden,
- Bewerten (E6) der Persistenz jedes Bereichs von Interesse auf den verschiedenen erhaltenen morphologischen Bildern, so dass der Bereich von Interesse morphologisch validiert wird, wobei die Bewertung der Persistenz darin besteht zu bewerten, ob sich Bereiche von Interesse auf mehreren morphologischen Bildern im gleichen Bereich befinden.

2. Verfahren nach Anspruch 1, wobei die morphologische Verarbeitung (E5) darin besteht, die Solidität eines oder mehrerer Pixelbereiche des Bildes zu bestimmen, wobei ein Bereich von Interesse identifiziert wird, wenn die zugeordnete Solidität über einem Schwellenwert liegt.

3. Verarbeitungsverfahren nach einem der vorstehenden Ansprüche, wobei mindestens N=2 Gruppen, vorzugsweise mindestens N=3 Gruppen von Ai > 1 Werten, $1 \leq i \leq N$, des charakteristischen Parameters (pcfib) des von den Signalen durchquerten Mediums betrachtet werden.

4. Verfahren nach Anspruch 3, wobei sich die Gruppen hinsichtlich der Schwankungsbereiche und/oder der Werte ganz oder teilweise überschneiden.

5. Verfahren nach einem der vorstehenden Ansprüche, wobei die Bewertung der Persistenz darin besteht, einen Prozentsatz der Präsenz eines Bereichs von Interesse auf den morphologischen Bildern zu bestimmen, wobei ein Bereich von Interesse für einen Prozentsatz oberhalb eines Schwellenwerts validiert wird.

6. Verfahren nach dem vorstehenden Anspruch, wobei das Sondennetzwerk K > 1 Sonden umfasst, die um den abzubildenden Bereich herum angeordnet sind, wobei das Netzwerk in vertikalen Positionen um den abzubildenden Bereich herum beweglich ist, wobei jede Konfiguration ein Sondenwinkelbereich ist, der sich jeweils aus M > 1 Sonden mit M < K zusammensetzt, wobei jede Konfiguration um mindestens eine Sonde relativ zu einer anderen Konfiguration winklig versetzt ist; für jeden Winkelbereich jede der Sonden der Reihe nach derart sendet, dass die für die Konfiguration erfassten Signale erzeugt werden, wobei ein elementares Radarbild für jeden Winkelbereich pro Gruppe charakteristischer Parameterwerte (pcfib) bestimmt wird.

7. Computerprogrammprodukt, das Programmcodeanweisungen für die Ausführung der Schritte des Verfahrens nach einem der Ansprüche 1 bis 6 umfasst, wenn dieses Verfahren von mindestens einem Prozessor ausgeführt wird.

8. Medizinische Bildgebungsvorrichtung, die eine Verarbeitungseinheit umfasst, die zur Durchführung eines Verfahrens nach einem der Ansprüche 1 bis 6 ausgelegt ist.

## Claims

1. A method for processing medical images of human tissues of an area of the body of a patient and particularly of the breast by means of a medical imaging device (1) comprising an array of probes emitting/receiving electromagnetic waves in the microwave range consisting of K > 1 probes spaced from each other, the array comprising P > 1 different configurations defining emitting probes and receiving probes for one or several positions around the area, in which the emitting probes are configured to emit microwave signals so as to illuminate an area of the body and the receiving probes are configured to receive microwave signals after diffusion and reflection into the area, the probes being able, in a complementary manner, to be configured to emit and receive simultaneously, the method comprising the following steps implemented in a processing unit of the medical imaging device:

- acquiring (E1) the signals produced in P > 1 configurations of the array of antennas;
- the method comprising for each configuration:

  - processing (E2) the signals acquired for N > 1 sets of Ai > 1 values, $1 \leq i \leq N$, of a parameter (pcfib) characteristic of dielectrically properties of the human tissues traversed by the signals, so as to obtain $\sum_{i=1}^{N} Ai$ elementary microwave radar images;
  - selecting (E3) in each of the N sets according to at least one image focusing criterion, an elementary microwave radar image, each selected elementary image corresponding to one of the values of the parameter (pcfib) of the set; one elementary image per set being selected for a configuration;

- the method comprising for each set:

  - reconstructing (E4), from the selected elementary radar images, each of the configurations of a radar image of the body area of a patient so as to reconstruct one 3D radar image per set;
  - morphologically processing (E5) each reconstructed radar image so as to obtain a morphological image on which one or several regions of interest, if existing, that may constitute a lesion, are identified,

- assessing (E6) the persistence of each region of interest on the different morphological images obtained in order to morphologically validate the region of interest, the assessing of the persistence consisting in evaluating whether regions of interest are present on several morphological images in a same area.

2. The method according to claim 1, wherein the morphological processing (E5) consists in determining the solidity of one or several pixel regions of the image, a region of interest being identified if the associated solidity is greater than a threshold.

3. The method according to any of the preceding claims, wherein at least N=2 sets are considered, preferably at least N=3 sets of Ai > 1 values, $1 \leq i \leq N$, of characteristic parameter (pcfib) of the medium traversed by the signals.

4. The method according to claim 3, wherein the sets totally or partially overlap in terms of variation ranges and/or in terms of values.

5. The method according to any of the preceding claims, wherein the assessment of the persistence consists in determining a percentage of presence of a region of interest on the morphological images, a region of interest being validated for a percentage greater than a threshold.

6. The method according to the preceding claim, wherein the array of probes comprises K > 1 probes disposed around the area to be imaged, said array being movable in vertical positions around the area to be imaged, each configuration being an angular sector of probes each composed of M > 1 probes with M < K, each configuration being angularly offset by at least one probe relative to another configuration; for each angular sector, each of the probes is in emission alternately so as to produce the signals acquired for the configuration, an elementary radar image being determined for each angular sector per set of characteristic parameter values (pcfib).

7. A computer program product comprising program code instructions for the execution of the steps of the method according to any of claims 1 to 6, when this method is executed by at least one processor.

8. A medical imaging device comprising a processing unit configured to implement a method according to any of claims 1 to 6.

# FIG. 1

# FIG. 2

# FIG. 3

Pcfib : [10:60]%

ROI

**FIG. 4a**
Pcfib : [10:60]%

**FIG. 4b**
Pcfib : [20:50]%

**FIG. 4c**
Pcfib : [10:20]%

**FIG. 4d**
Pcfib : [30:40]%

**FIG. 4e**
Pcfib : [50:60]%

**FIG. 5b**
Pcfib : [20:50]%

**FIG. 5d**
Pcfib : [50:60]%

**FIG. 5a**
Pcfib : [10:60]%

**FIG. 5c**
Pcfib : [30:40]%

## RÉFÉRENCES CITÉES DANS LA DESCRIPTION

**Documents brevets cités dans la description**

- US 2013018591 A1, GRZEGORCZYK TOMASZ M
- CN 111067524 A
- US 2005107692 A1, LI JIAN
- US 2019175095 A1, BORE CHRIS

**Littérature non-brevet citée dans la description**

- Super-resolution radar imaging for breast cancer detection with microwaves: the integrated information selection criteria. **FASOULA A.** 2019 41ST ANNUAL INTERNATIONAL CONFERENCE OF THE IEEE ENGINEERING IN MEDICINE AND BIOLOGY SOCIETY (EMBC). IEEE, 23 July 2019, 1868-1874
- **A.J. DEVANEY**. Time reversal imaging of obscured targets from multistatic data. *IEEE Trans. Antennas Propag.*, 2005
- **MARENGO, E.A.** ; **GRUBER, F.K.** ; **SIMONETTI, F.** Time-reversal MUSIC imaging of extended targets. *IEEE Trans. Image Process.*, 2007, vol. 16, 1967-1984
- **HOSSAIN, M.D.** ; **MOHAN, A.S.** Cancer Detection in Highly Dense Breasts Using Coherently Focused Time-Reversal Microwave Imaging. *IEEE Trans. Comput. Imaging*, 2017, vol. 3, 928-939
- **A. FASOULA** ; **B.M. MOLONEY** ; **L. DUCHESNE** ; **J.D.G. CANO** ; **B.L. OLIVEIRA** ; **J. BERNARD** ; **M.J. KERIN**. Super-resolution radar imaging for breast cancer detection with microwaves: the integrated information selection criteria. *41st Annu. Int. Conf. IEEE Eng. Med. Biol. Soc.*, 2019
- **T. SUGITANI** ; **S.I. KUBOTA** ; **S.I. KUROKI** ; **K. SOGO** ; **K. ARIHIRO** ; **M. OKADA** ; **T. KADOYA** ; **M. HIDE** ; **M. ODA** ; **T. KIKKAWA**. Complex permittivities of breast tumor tissues obtained from cancer surgeries. *Appl. Phys. Lett.*, 2014
- **M. LAZEBNIK** ; **L. MCCARTNEY** ; **D. POPOVIC** ; **C.B. WATKINS** ; **M.J. LINDSTROM** ; **J. HARTER** ; **S. SEWALL** ; **A. MAGLIOCCO** ; **J.H. BOOSKE** ; **M. OKONIEWSKI**. A large-scale study of the ultrawideband microwave dielectric properties of normal breast tissue obtained from reduction surgeries. *Phys. Med. Biol.*, 2007
- **S. PERTUZ** ; **D. PUIG** ; **M.A. GARCIA**. Analysis of focus measure operators for shape-from-focus. *Pattern Recognit.*, 2013
- **O'LOUGHLIN, D.** ; **KREWER, F.** ; **GLAVIN, M.** ; **JONES, E.** ; **O'HALLORAN, M.** Focal quality metrics for the objective evaluation of confocal microwave images. *Int. J. Microw. Wirel. Technol.*, 2017, vol. 9, 1365-1372
- **T.F. DE BRITO SILVA** ; **A.C. DE PAIVA** ; **A.C. SILVA** ; **G. BRAZ JÚNIOR** ; **J.D.S. DE ALMEIDA**. Classification of breast masses in mammograms using geometric and topological feature maps and shape distribution. *Res. Biomed. Eng.*, 2020
- **N. SAFDARIAN** ; **M. HEDYEZADEH**. Detection and Classification of Breast Cancer in Mammography Images Using Pattern Recognition Methods. *Multidiscip. Cancer Investig.*, 2019